(19) Europäisches Patentamt
European Patent Office
Office européen des brevets

(11) Veröffentlichungsnummer : **0 064 193**
**B1**

(12) # EUROPÄISCHE PATENTSCHRIFT

(45) Veröffentlichungstag der Patentschrift : **25.07.84**

(51) Int. Cl.[3] : **C 07 C 25/18**, C 07 C 43/192, C 09 K 3/34, G 02 F 1/13

(21) Anmeldenummer : **82103206.7**

(22) Anmeldetag : **16.04.82**

(54) **Fluorhaltige 4,4'-Bis-(cyclohexyl)-biphenylderivate, diese enthaltende Dielektrika und elektrooptisches Anzeigeelement.**

(30) Priorität : **30.04.81 DE 3117152**

(43) Veröffentlichungstag der Anmeldung : **10.11.82 Patentblatt 82/45**

(45) Bekanntmachung des Hinweises auf die Patenterteilung : **25.07.84 Patentblatt 84/30**

(84) Benannte Vertragsstaaten : **AT CH DE FR GB IT LI NL**

(56) Entgegenhaltungen :
**EP-A- 0 030 761**
**DE-A- 2 636 684**

(73) Patentinhaber : **Merck Patent Gesellschaft mit beschränkter Haftung**
**Frankfurter Strasse 250**
**D-6100 Darmstadt (DE)**

(72) Erfinder : **Eidenschink, Rudolf, Dr.**
**Erlenweg 17**
**D-6110 Dieburg (DE)**
Erfinder : **Römer, Michael, Dr.**
**Im Grossen Garten 18**
**D-6054 Rodgau 2 (DE)**
Erfinder : **Pohl, Ludwig, Dr.**
**Niebergallweg 5**
**D-6100 Darmstadt (DE)**

**EP 0 064 193 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Für elektrooptische Anzeigeelemente werden in großem Umfang die Eigenschaften nematischer oder nematischcholesterischer flüssigkristalliner Materialien ausgenutzt, ihre optischen Eigenschaften wie Lichtabsorption, Lichtstreuung, Doppelbrechung, Reflexionsvermögen oder Farbe unter dem Einfluß elektrischer Felder signifikant zu verändern. Die Funktion derartiger Anzeigeelemente beruht dabei beispielsweise auf den Phänomenen der dynamischen Streuung, der Deformation aufgerichteter Phasen, auf dem Schadt-Helfrich-Effekt in der verdrillten Zelle oder auf dem cholesterisch-nematischen Phasenübergang.

Für die technische Anwendung dieser Effekte in elektronischen Bauelementen werden flüssigkristalline Dielektrika benötigt, die einer Vielzahl von Anforderungen genügen müssen. Besonders wichtig sind hier die chemische Beständigkeit gegenüber Feuchtigkeit, Luft und physikalischen Einflüssen wie Wärme, Strahlung im infraroten, sichtbaren und ultravioletten Bereich und elektrische Gleich- und Wechselfelder. Ferner wird von technisch verwendbaren flüssigkristallinen Dielektrika eine flüssigkristalline Mesophase im Temperaturbereich von mindestens + 10 °C bis + 50 °C, bevorzugt von 0 °C bis 60 °C, und eine möglichst niedrige Viskosität bei Raumtemperatur, die vorzugsweise nicht mehr als $70 \cdot 10^{-3}$ Pa.s betragen soll, gefordert. Schließlich dürfen sie im Bereich des sichtbaren Lichtes keine Eigenabsorption aufweisen, d. h. sie müssen farblos sein.

Es ist bereits eine Anzahl von flüssigkristallinen Verbindungen bekannt, die den an Dielektrika für elektronische Bauelemente gestellten Stabilitätsanforderungen genügen und auch farblos sind. Hierzu gehören insbesondere die in der DE-OS 2 139 628 beschriebenen p,p'-disubstituierten Benzoesäurephenylester und die in der DE-OS 2 636 684 beschriebenen p,p'-disubstituierten Phenylcyclohexanderivate. In beiden genannten Verbindungsklassen wie auch in anderen bekannten Reihen von Verbindungen mit flüssigkristalliner Mesophase gibt es keine Einzelverbindungen, die in dem geforderten Temperaturbereich von 10 °C bis 60 °C eine flüssigkristalline nematische Mesophase ausbilden. Es werden daher in der Regel Mischungen von zwei oder mehreren Verbindungen hergestellt, um als flüssigkristalline Dielektrika verwendbare Substanzen zu erhalten. Hierzu mischt man gewöhnlich mindestens eine Verbindung mit niedrigem Schmelz- und Klärpunkt mit einer anderen mit deutlich höherem Schmelz- und Klärpunkt. Hierbei wird normalerweise ein Gemisch erhalten, dessen Schmelzpunkt unter dem der niedriger schmelzenden Komponente liegt, während der Klärpunkt zwischen den Klärpunkten der Komponenten liegt. Trotzdem bereitet die Herstellung optimaler Dielektrika immer wieder Schwierigkeiten, da die Komponenten mit den hohen Schmelz- und Klärpunkten den Gemischen häufig auch eine hohe Viskosität verleihen. Dadurch werden die Schaltzeiten der damit hergestellten elektrooptischen Anzeigeelemente in unerwünschter Weise verlängert. Ferner treten oft Probleme dadurch auf, daß die Löslichkeit der verschiedenen Komponenten ineinander, insbesondere bei Raumtemperatur oder tieferen Temperaturen nur sehr begrenzt ist.

Der Erfindung liegt die Aufgabe zugrunde, flüssigkristalline Dielektrika herzustellen, die eine nematische Phase im geforderten Temperaturbereich aufweisen und in Flüssigkristallzellen bei Raumtemperatur ausreichend kurze Schaltzeiten ermöglichen.

Es wurde nun gefunden, daß die 4,4'-Bis-(cyclohexyl)-biphenylderivate der Formel (I)

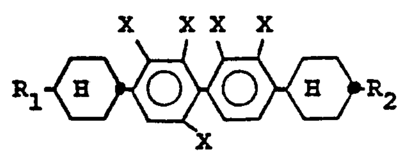

(I)

worin $R_1$ und $R_2$, die gleich oder verschieden sind, Alkyl oder Alkoxy mit bis zu 8 C-Atomen, und einer oder zwei der Substituenten X Fluor und die übrigen Wasserstoff bedeuten,

vorzüglich als Komponenten flüssigkristalliner Dielektrika geeignet sind. Dabei besitzen diese Verbindungen einen außerordentlichen breiten Anwendungsbereich : in Abhängigkeit von der Auswahl der Substituenten können die Verbindungen der Formel (I) sowohl als Basismaterialien dienen, aus denen flüssigkristalline Dielektrika zum überwiegenden Teil bestehen, es können aber auch Verbindungen der Formel (I) in geringeren Anteilen von beispielsweise 2 bis 45 Gewichtsprozent flüssigkristallinen Basismaterialien aus anderen Verbindungsklassen zugesetzt werden, um so Dielektrika mit einer verbreiterten flüssigkristallinen Mesophase herzustellen oder die Größe der dielektrischen Anisotropie eines solchen Dielektrikums zu beeinflussen.

Durch geeignete Auswahl der Substituenten $R_1$ und $R_2$ sowie der bzw. den Positionen der Fluoratome lassen sich mit den Verbindungen der Formel (I) sowohl Dielektrika mit positiver dielektrischer Anisotropie zur Verwendung in Anzeigeelementen auf der Basis der verdrillten nematischen Zelle oder des cholesterisch-nematischen Phasenübergangs herstellen, es können aber auch Dielektrika mit von Null nur wenig verschiedener oder auch mit negativer dielektrischer Anisotropie hergestellt werden,

die in Anzeigeelementen auf der Basis der dynamischen Streuung oder der Deformation aufgerichteter Phasen (DAP-Effekt) verwendet werden.

Die Verbindungen der Formel (I) sind in reinem Zustand farblos, und bilden niederviskose nematische Mesophasen in einem erstaunlich breiten und für die elektrooptische Verwendung günstig gelegenen Temperaturbereich.

Gegenstand der Erfindung sind somit die 4,4'-Bis-(cyclohexyl)-biphenylderivate der Formel (I) und ihre Verwendung als Komponenten flüssigkristalliner Dielektrika. Gegenstand der Erfindung sind weiterhin flüssigkristalline Dielektrika mit einem Gehalt an mindestens einem 4,4'-Bis-(cyclohexyl)-biphenylderivat der Formel (I) sowie elektrooptische Anzeigeelemente auf der Basis einer Flüssigkristallzelle, die ein derartiges flüssigkristallines Dielektrikum enthalten.

In den erfindungsgemäßen 4,4'-Bis-(cyclohexyl)-biphenyl-derivaten der Formel (I) ist der Biphenylteil durch ein oder zwei Fluoratome substituiert. Wenn nur ein Fluoratom im Molekül enthalten ist, sind die Verbindungen der Formel (Ia) bevorzugt, in denen das Fluoratom in einer ortho-Position zu der Bindung zwischen den beiden Phenylringen steht.

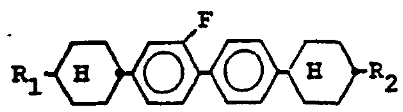

(Ia)

Wenn die Verbindungen der Formel (I) zwei Fluoratome enthalten, sind darunter ebenfalls diejenigen bevorzugt, in denen die Fluoratome in ortho-Positionen zu der Bindung zwischen den beiden Phenylringen angeortnet sind, das sind die Verbindungen der Formeln (Ib) und (Ic) :

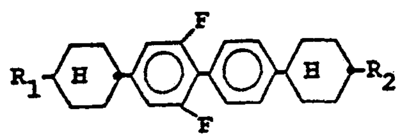

(Ib)

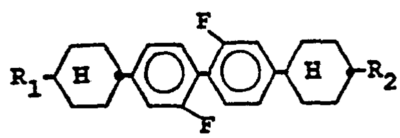

(Ic)

$R_1$ und $R_2$ haben in diesen Teilformeln die gleiche Bedeutung wie in der Formel (I). Die Substituenten in den 1- und 4-Positionen der Cyclohexanringe sind immer trans- ständig angeordnet ; dies ist in den Formelbildern durch den verstärkten schwarzen Punkt an der rechten Seite der Cyclohexanringe kenntlich gemacht.

Die nicht von den Teilformeln (Ia) bis (Ic) umfaßten Verbindungen der Formel (I) besitzen zwar die gleichen vorteilhaften Eigenschaften wie die der herausgehobenen Gruppen, jedoch ist ihre Herstellung schwieriger und daher weniger wirtschaftlich ; die Verbindungen der Teilformeln (Ia) bis (Ic) sind daher bevorzugt.

In den Verbindungen der Formel (I) können die Alkyl- bzw. Alkoxyreste $R_1$ und $R_2$ geradkettig oder verzweigt sein. Wenn sie geradkettig sind, also Methyl, Ethyl, n-Propyl, n-Butyl, n-Pentyl, n-Hexyl, n-Heptyl oder n-Octyl, bzw. die entsprechenden Alkoxygruppen bedeuten, besitzen die dadurch charakterisierten Verbindungen in der Regel höhere Klärpunkte als die mit verzweigten Flügelgruppen $R_1$ und/oder $R_2$. Deswegen enthält gewöhnlich höchstens eine der Flügelgruppen $R_1$ und $R_2$ eine verzweigte Kohlenstoffkette. Verbindungen der Formel (I) mit einer verzweigten Flügelgruppe $R_1$ oder $R_2$ sind gelegentlich wegen einer besseren Löslichkeit in den üblichen flüssigkristallinen Basismaterialien von Bedeutung, insbesondere aber als chirale Dotierstoffe, wenn sie durch die Kettenverzweigung optische Aktivität besitzen. Solche verzweigten Flügelgruppen enthalten nicht mehr als eine Kettenverzweigung. Bevorzugte verzweigte Kohlenwasserstoffreste sind die, in denen sich an einer längeren Kohlenstoffkette in 1-, 2- oder 3-Stellung eine Methyl- oder Ethylgruppe befindet, beispielsweise 2-Methylpropyl, 2-Methylbutyl, 3-Methylbutyl, 2-Methylpentyl, 3-Methylpentyl, 2-Ethylhexyl oder 1-Methylhexyl. Zusammen können die Flügelgruppen $R_1$ und $R_2$ bis zu 16 Kohlenstoffatome enthalten. Im Rahmen der vorliegenden Erfindung sind darunter diejenigen bevorzugt, in denen $E_1$ und $R_2$ zusammen 3 bis 14, insbesondere von 4 bis zu 12 Kohlenstoffatomen enthalten. Vorzugsweise ist mindestens eine der Flügelgruppen eine Alkylgruppe.

Die erfindungsgemäßen Verbindungen werden in für derartige Substanzen üblicher Weise hergestellt. Ein bevorzugtes Verfahren besteht darin, daß Verbindungen der Formel (II),

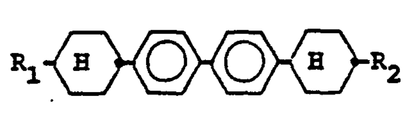

(II)

worin $R_1$ und $R_2$ die bei Formel (I) angegebene Bedeutung besitzen, mit einem Nitrierungsmittel behandelt werden, die dabei entstehenden Mono- und Dinitro-4,4'-bis-(cyclohexyl)-biphenyle in an sich üblicher Weise abgetrennt werden und zu den entsprechenden Mono- und Diaminoverbindungen reduziert werden ; diese Aminoverbindungen werden dann diazotiert, durch Umsetzung mit einem Fluoborat in die entsprechenden Diazoniumfluoborate überführt und daraus durch thermische Zersetzung die fluorhaltigen 4,4'-Bis-(cyclohexyl)-biphenyle der Formel (I) hergestellt. Soweit dabei Gemische der Mono- und Difluorverbindungen bzw. Isomeren erhalten werden, werden diese dann in an sich üblicher Weise, zum Beispiel durch fraktionierte Kristallisation, Extraktion oder auf chromatographischem Wege getrennt. Selbstverständlich kann eine derartige Trennung auch bereits auf eine der vorhergehenden Synthesestufen, zum Beispiel nach der Nitrierung oder im Anschluß an die Reduktion oder an die Herstellung der Diazoniumtetrafluoborate erfolgen.

Die Nitrierung der Ausgangsmaterialien der Formel (II) erfolgt in an sich bekannter Weise, zum Beispiel mit einem Gemisch von Schwefelsäure und Salpetersäure, mit Essigsäure/Salpetersäure oder mit einem Acylnitrat wie Benzoylnitrat oder Acetylnitrat. Durch geeignete Wahl der an sich aus der Literatur bekannten Nitrierungsbedingungen, z. B. Art und Konzentration des nitrierenden Agens, Lösungsmittel, Temperatur, Reaktionsdauer und/oder Katalysator, kann die Isomerenverteilung in dem entstehenden Gemisch von Nitrierungsprodukten in Richtung auf die hauptsächlich gewünschten Produkte beeinflußt werden. Die Reduktion der Nitroverbindungen zu den Aminoverbindungen wird nach Standardmethoden durchgeführt, zum Beispiel durch katalytische Hydrierung, durch Behandlung mit Natriumsulfid, mit wäßrigem Dithionit oder mit Zinn-(II)-chlorid und Salzsäure.

Die Diazotierung, Umsetzung zum Diazoniumtetrafluoborat und thermische Zersetzung (Schiemann-Balz-Synthese) werden gleichfalls in an sich bekannter Weise durchgeführt, zum Beispiel nach einer der in « Organic Reactions », Band 5 (1949), Seiten 193-228 beschriebenen Verfahrensvarianten.

Bei einem anderen Syntheseverfahren wird ein ein- oder zweifach durch Fluor substituiertes 4-Brombiphenyl als Ausgangsmaterial verwendet. Dies wird nacheinander mit Magnesium, einem 4-Alkyl- bzw. -Alkoxycyclohexanon und einer Säure umgesetzt, und dann das so erhaltene 4-[4-Alkyl- oder -Alkoxycyclohexen-1-yl]-mono- oder -difluorbiphenyl zum 4-(4-Alkyl- oder -Alkoxycyclohexyl)-mono- oder -difluorbiphenyl hydriert. Dieses Zwischenprodukt wird bromiert und in dem erhaltenen 4'-Brom-4-(4-Alkyl- oder -Alkoxycyclohexyl)-mono- oder -difluorbiphenyl das Bromatom auf die vorstehend beschriebene Weise gegen eine 4-Alkyl- oder -Alkoxycyclohexylgruppe ausgetauscht. Anstelle der Umsetzung mit Magnesium zur intermediären Grignard-Verbindung können die Bromatome in den Zwischenprodukten auch durch Behandlung mit einer metallorganischen Verbindung, zum Beispiel Butyllithium, und anschließende Umsetzung mit den Cyclohexanonderivaten gegen die Cyclohexylgruppen ausgetauscht werden. Soweit dabei Gemische von cis- und trans-Cyclohexylderivaten entstehen, können diese in an sich üblicher Weise, zum Beispiel durch Behandlung mit Kalium-tert-butylat in Dimethylformamid in die thermodynamisch stabileren trans-Verbindungen umgewandelt werden. Eventuell im Gleichgewichtsgemisch noch enthaltene Anteile der unerwünschten Cis-Isomeren können anschließend durch fraktionierte Kristallisation oder mit chromatografischen Methoden abgetrennt werden.

Die erfindungsgemäßen Dielektrika bestehen aus 2 bis 15, vorzugsweise 3 bis 12 Komponenten, darunter mindestens einem fluorhaltigen 4,4'-Bis-(cyclohexyl)-biphenylderivat der Formel (I). Die anderen Bestandteile werden ausgewählt aus den nematischen oder nematogenen Substanzen aus den Klassen der Azoxybenzole, Benzylidenaniline, Biphenyle, Terphenyle, Phenyl- oder Cyclohexylbenzoate, Cyclohexancarbonsäure-phenyl- oder -cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexyldioxane, ggf. halogenierte Stilbene, Benzylphenylether, Tolane und substituierten Zimtsäuren. Die wichtigsten als Bestandteile derartiger flüssigkristalliner Dielektrika in Frage kommenden Verbindungen lassen sich durch die Formel (III) charakterisieren.

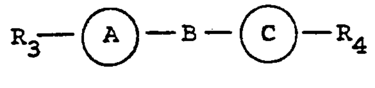

(III)

worin A und C je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituierten Naphthalin, Di- und Tetrahydronaphthalin, Chinazolin und Tetrahydrochinazolin gebildeten Gruppe,

B —CH=CH— —N(O)=N—
—CH=CY— —CH=N(O)—
—C≡C— $—CH_2—CH_2—$
—CO—O— $—CH_2—O—$
—CO—S— $—CH_2—S—$

—CH=N— 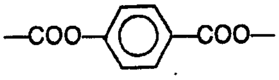

oder eine C—C— Einfachbindung,

Y Halogen, vorzugsweise Chlor, oder —CN, und $R_3$ und $R_4$ Alkyl, Alkoxy, Alkanoyloxy oder Alkoxycarbonyloxy mit bit zu 18, vorzugsweise bis zu 8 Kohlenstoffatomen, oder einer dieser Reste auch —CN, —NC, —$NO_2$, —$CF_3$, F, Cl oder Br bedeuten. Bei den meisten dieser Verbindungen sind $R_3$ und $R_4$ voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich.

Die erfindungsgemäßen Dielektrika enthalten in der Regel mindestens 30, vorzugsweise 50-99, insbesondere 60-98 Gewichtsteile der Verbindungen der Formel (I) und (III). Hiervon entfallen bevorzugt mindestens 5 Gewichtsteile, meist auch 10 oder mehr Gewichtsteile auf eine oder mehrere Verbindungen der Formel (I). Jedoch werden von der Erfindung auch solche flüssigkristallinen Dielektrika umfaßt, denen beispielsweise zu Dotierungszwecken nur weniger als 5 Gewichtsteile, zum Beispiel 0,1 bis 3 Gewichtsteile einer oder mehrerer Verbindungen der Formel (I) zugesetzt worden sind. Andererseits können die Verbindungen der Formel (I) bis zu 60 Gewichtsprozent der erfindungsgemäßen Dielektrika ausmachen. Vorzugsweise enthalten die flüssigkristallinen Dielektrika nach der Erfindung 10 bis 30 Gewichtsprozent einer oder mehrerer Verbindungen der Formel (I).

Die Herstellung der erfindungsgemäßen Dielektrika erfolgt in an sich üblicher Weise. In der Regel wird die gewünschte Menge der in geringerer Menge verwendeten Komponenten in der den Hauptbestandteil ausmachenden Komponente gelöst, zweckmäßig bei erhöhter Temperatur. Wenn dabei eine Temperatur oberhald des Klärpunkts des Hauptbestandteils gewählt wird, kann die Vollständigkeit des Lösevorgangs besonders leicht beobachtet werden.

Durch geeignete Zusätze können die flüssigkristallinen Dielektrika nach der Erfindung so modifiziert werden, daß sie in allen bisher bekannt gewordenen Arten von Flüssigkristallanzeigeelementen verwendet werden können. Derartige Zusätze sind dem Fachmann bekannt und sind in der einschlägigen Literatur ausführlich beschrieben. Beispielsweise können dichroitische Farbstoffe oder Substanzen zur Veränderung der dielektrischen Anisotropie, der Viskosität, der Leitfähigkeit und/oder der Orientierung der nematischen Phasen zugesetzt werden. Derartige Substanzen sind zum Beispiel in den DE-OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. In den Beispielen bedeuten F. den Schmelzpunkt und K. den Klärpunkt einer flüssigkristallinen Substanz in Grad Celsius ; Siedetemperaturen sind mit Kp. bezeichnet. Wenn nichts anderes angegeben ist, bedeuten Angaben von Teilen oder Prozent Gewichtsteile bzw. Gewichtsprozent.

## Beispiel 1

a) 45,8 g 4,4'-Bis(trans-4-n-pentylcyclohexyl)-biphenyl werden in eine 40° warme Mischung aus 20 ml 65 %iger Salpetersäure und 24 ml 96 %iger Schwefelsäure eingetragen. Nach beendeter Zugabe wird das Reaktionsgemisch noch 1 Std. bei 60° gerührt und auf 300 g Eis gegossen. Das auskristallisierte 4,4'-Bis-(trans-4-n-pentylcyclohexyl)-2-nitrobiphenyl wird abfiltriert und aus Äthanol umkristallisiert.

b) 20 g 4,4'-Bis(trans-4-n-pentylcyclohexyl)-2-nitrobiphenyl werden in 150 ml Tetrahydrofuran gelöst. Nach Zugabe von 3 g Palladium-Kohle (10 % Pd) wird 1 Std. lang bei Normaldruck und Raumtemperatur Wasserstoff eingleitet. Anschließend wird vom Katalysator abfiltriert und das Filtrat eingedampft. Das zurückbleibende 4,4'-Bis-(trans-4-n-pentylcyclohexyl)-2-aminobiphenyl wird aus Petrolether (Siedebereich 40-60°) umkristallisiert.

c) 15,0 g 4,4'-Bis(trans-4-n-pentylcyclohexyl)-2-aminobiphenyl werden in 10 ml 36 %iger wässriger Salzsäure aufgeschlämmt. Nach Zugabe von 10 ml Dioxan wird bei 0 °C die Lösung von 3,8 g Natriumnitrit in 15 ml Wasser zugetropft. Unmittelbar darauf wird, ebenfalls bei 0 °C eine Lösung von 12 g Natriumtetrafluoroborat in 20 ml Wasser zugetropft. Der sich bildende Niederschlag wird nache 30 Minuten abfiltriert, mit Eiswasser gewaschen und unter vermindertem Druck bei Raumtemperatur getrocknet ; das pulvertrockne Diazoniumtetrafluoborat wird bis zum Ende der Gasentwicklung auf 120° erhitzt und das zurückbleibende 4,4'-Bis-(trans-4-n-pentylcyclohexyl)-2-Fluorbiphenyl aus Ethanol umkristallisiert. Analog werden hergestellt :

4,4'-Bis-(trans-4-n-propylcyclohexyl)-2-fluorbiphenyl,
4-(trans-4-Methylcyclohexyl)-4'-(trans-4-n-butylcyclohexyl-2-fluorbiphenyl,
4-(trans-4-Ethylcyclohexyl)-4'-(trans-4-n-propylcyclohexyl)-2-fluorbiphenyl,
4-(trans-4-n-Propylcyclohexyl)-4'-(trans-4-n-pentylcyclohexyl)-2-fluorbiphenyl,
4-(trans-4-n-Pentylcyclohexyl)-4'-(trans-4-n-propylcyclohexyl)-2-fluorbiphenyl,
4-(trans-4-Ethoxycyclohexyl)-4'-(trans-4-n-propylcyclohexyl)-2-fluorbiphenyl,
4-(trans-4-n-Hexylcyclohexyl)-4'-(trans-4-n-butyloxycyclohexyl)-2-fluorbiphenyl,
4-(trans-4-n-Heptylcyclohexyl)-4'-(trans-methylcyclohexyl)-2-fluorbiphenyl,
4-(trans-4-Methoxycyclohexyl)-4'-[trans-4-(2-methylbutyl)-cyclohexyl)]-2-fluorbiphenyl,
4,4'-Bis-(trans-4-n-Butylcyclohexyl)-2-fluorbiphenyl,

4,4'-Bis-(trans-4-Ethylcyclohexyl)-2-fluorbiphenyl,
4,4'-Bis-(trans-4-n-Propylcyclohexyl)-2,5-difluorbiphenyl.

Beispiel 2

a) Eine Lösung von 24,5 g 4-(trans-4-n-Pentylcyclohexyl)-anilin in 100 ml Toluol wird nacheinander mit 35 ml Pyridin und dann tropfenweise mit einer Lösung von 12 g Acetylchlorid in 25 ml Toluol versetzt. Anschließend wird das Reaktionsgemisch mit 100 ml Wasser ausgeschüttelt, die organische Phase abgetrennt, über Natriumsulfat getrocknet und eingedampft. 20 g des zurückbleibenden Acetyl-N-4-(trans-4-n-pentylcyclohexyl)-anilids werden in Portionen von 1-2 g in ein Gemisch aus 118 ml Salpetersäure (d = 1,40) und 47 ml Schwefelsäure (d = 1,84) so eingetragen, daß die Temperatur zwischen 30 °C und 40° bleibt. Nach beendeter Zugabe wird noch 15 Minuten gerührt und dann in 700 ml kaltes Wasser gegossen. Das Zweiphasensystem wird mit Dichlormethan extrahiert, wonach die organische Phase durch Destillation vom Lösungsmittel befreit wird. Der Rückstand wird in 100 ml siedendem Ethanol gelöst, danach wird eine Lösung von 15 g Kaliumhydroxid in 20 ml Wasser zugesetzt und noch 20 Minuten am Rückfluß erhitzt. Nach dem Eingießen in 500 ml Wasser wird wiederum mit Dichlormethan extrahiert, das Lösungsmittel abdestilliert und das zurückbleibende 4-(trans-4-n-Pentylcyclohexyl)-2-nitroanilin durch Destillation unter vermindertem Druck gereinigt ; $Kp._{0,3mbar}$ 139-143°.

b) 12,0 g 4-(trans-4-n-pentylcyclohexyl)-2-nitroanilin werden in 10 ml 36 %iger Salzsäure aufgeschlämmt. Nach Zugabe von 10 ml Dioxan wird bei 0 °C eine Lösung von 2,8 g Natriumnitrit in 6 ml Wasser zugetropft. Hierauf läßt man unter starkem Rühren eine Lösung von 4,5 g Kupfer(I)-chlorid in 17 ml 36 %iger Salzäure einfließen. Nach eintündigem Rühren wird auf Raumtemperatur erwärmt, das Reaktionsgemisch in 200 ml Wasser gegossen und mit Dichlormethan extrahiert. Die organische Phase wird über Calciumchlorid getrocknet, eingedampft und das zurückbleibende 4,4'-Bis-(trans-4-n-pentylcyclohexyl)-2-2'-dinitrobiphenyl aus Ethanol umkristallisiert.

c) Zu einer Lösung von 7,5 g 4,4'-Bis(trans-4-n-pentylcyclohexyl)-2,2'-dinitrobiphenyl in 100 ml Tetrahydrofuran werden 2 g Palladium-Kohle (10 % Pd) gegeben und in diese Suspension 1 Std. lang bei Raumtemperatur Wasserstoff eingleitet. Anschließend wird vom Katalysator abfiltriert und das Filtrat eingedampft. 4,0 g des zurückbleibenden 4,4'-Bis(trans-4-n-pentylcyclohexyl)-2,2'-diaminobiphenyls werden in 15 ml 26 %iger wäßriger Tetrafluorborsäure suspendiert. Nach Zugabe von 5 ml Dioxan wird bei 0 °C die Lösung von 1,2 g Natriumnitrit in 6 ml Wasser zugetropft. Der sich bildende Niederschlag wird nach 1-2 Stunden abfiltriert, mit Eiswasser gewaschen und bei Raumtemperatur im Vakuum getrocknet. Das getrocknete Pulver wird auf 120° erhitzt. Nach Beendigung der $BF_3$-Entwicklung wird das zurückbleibende 4,4'-Bis-(trans-4-n-pentylcyclohexyl)-2,2'-difluorbiphenyl umkristallisiert.

Analog werden hergestellt :

4,4'-Bis-(trans-4-ethylcyclohexyl)-2,2'-difluorbiphenyl,
4,4'-Bis-(trans-4-n-propylcyclohexyl)-2,2'-difluorbiphenyl,
4,4'-Bis-(trans-4-n-butylcyclohexyl)-2,2'-difluorbiphenyl,
4,4'-Bis-(trans-4-n-hexylcyclohexyl)-2,2'-difluorbiphenyl,
4,4'-Bis-(trans-4-ethoxycyclohexyl)-2,2'-difluorbiphenyl.

Beispiel 3

Eine flüssigkristalline Mischung aus

23,9 % 4-(trans-4-n-Propylhexyl)-benzonitril,
36,1 % 4-(trans-4-n-Pentylcyclohexyl)-benzonitril,
25,0 % 4-(trans-4-n-Heptylcyclohexyl)-benzonitril, und
15,0 % 4-(trans-4-n-Pentylcyclohexyl-4'-cyanobiphenyl

hat einen Schmelzpunkt von − 6°, einen Klärpunkt von + 70° und eine Viskosität von $29 \cdot 10^{-3}$ Pa · s bei 20°.

Ein flüssigkristallines Dielektrikum aus 90 % dieser Mischung und 10 % 4,4'-Bis-(trans-4-n-pentylcyclohexyl)-2-fluorbiphenyl hat einen Schmelzpunkt von − 15°, einen Klärpunkt von 86° und eine Viskosität von $27 \cdot 10^{-3}$ Pa · s bei 20°.

Beispiel 4

Eine flüssigkristalline Mischung aus

21,1 % 4-(trans-4-Ethylcyclohexyl)-benzonitril,
22,2 % 4-(trans-4-n-Butylcyclohexyl)-benzonitril,

13,9 % 4-Ethyl-4'-cyanobiphenyl,
17,8 % 4-n-Butyl-4'-cyanobiphenyl,
16,1 % 4-(trans-4-n-Pentylcyclohexyl)-4'-cyanobiphenyl, und
8,9 % 4-n-Pentyl-4''-cyano-p-terphenyl

hat eine nematische Phase im Temperaturbereich von − 6° bis + 64° und eine Viskosität von $32 \cdot 10^{-3}$ Pa · s bei 20°.

Ein Flüssigkristallines Dielektrikum aus 90 % dieser Mischung und 10 % 4,4'-Bis-(trans-4-n-pentylcyclohexyl)-2-fluorbiphenyl hat eine nematische Phase in dem erweiterten Temperaturbereich von − 12° bis + 80° und eine Viskosität von $33 \cdot 10^{-3}$ Pa · s bei 20°.

Beispiel 5

Die flüssigkristalline Mischung aus

38,2 % 4-(trans-4-n-Pentylcyclohexyl)-benzonitril,
15,3 % 4-(trans-5-n-Propyl-1,3-dioxanyl-2-)-benzonitril,
15,9 % 4-(trans-5-n-Pentyl-1,3-dioxanyl-2-)-benzonitril,
18,8 % 4-(trans-5-n-Hexyl-1,3-dioxanyl-2-)-benzonitril, und
11,8 % 4-(trans-4-n-Pentylcyclohexyl)-4'-cyanobiphenyl

hat eine nematische Mesophase im Temperaturbereich von − 5° bis + 62° und eine Viskosität von $35 \cdot 10^{-3}$ Pa · s bei 20°.

Ein flüssigkristallines Dielektrikum aus 85 % dieser Mischung und 15 % 4,4'-Bis-(trans-4-n-pentylcyclohexyl)-2-fluorbiphenyl hat eine nematische Phase in dem erweiterten Temperaturbereich von − 15° bis + 86° und eine Viskosität von $26 \cdot 10^{-3}$ Pa · s bei 20°.

**Ansprüche**

1. Verbindungen der Formel (I)

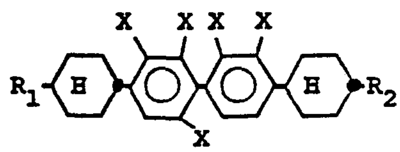

(I)

worin $R_1$ und $R_2$, die gleich oder verschieden sind, Alkyl oder Alkoxy mit bis zu 8 C-Atomen, und einer oder zwei der Substituenten X Fluor und die übrigen Wasserstoff bedeuten.

2. Verbindungen nach Anspruch 1 der Formel (Ia)

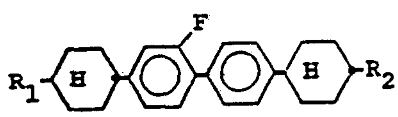

(Ia)

worin $R_1$ und $R_2$ gleich oder verschieden sind und Alkyl oder Alkoxy mit bis zu 8 C-Atomen bedeuten.

3. Verbindungen nach Anspruch 1 der Formel (Ib)

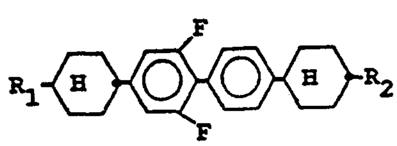

(Ib)

worin $R_1$ und $R_2$ gleich oder verschieden sind und Alkyl oder Alkoxy mit bis zu 8 C-Atomen bedeuten.

4. Verbindungen nach Anspruch 1 der Formel (Ic)

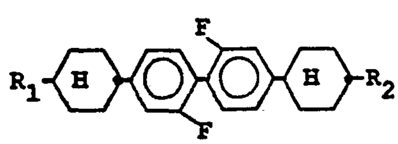

(Ic)

worin $R_1$ und $R_2$ gleich oder verschieden sind und Alkyl oder Alkoxy mit bis zu 8 C-Atomen bedeuten.

5. Verwendung der Verbindungen der Formel (I)

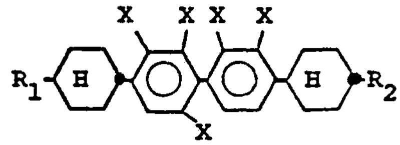

(I)

worin $R_1$ und $R_2$ gleich oder verschieden sind und Alkyl oder Alkoxy mit bis zu 8 C-Atomen, und einer oder zwei der Substituenten X Fluor und die übrigen Wasserstoff bedeuten,. als Komponenten flüssigkristalliner Dielektrika.

6. Flüssigkristallines Dielektrikum für elektrooptische Anzeigeelemente mit mindestens 2 flüssigkristallinen Komponenten, dadurch gekennzeichnet, daß mindestens eine dieser flüssigkristallinen Komponenten eine Verbindung der Formel (I) ist,

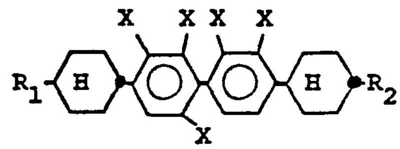

(I)

worin $R_1$ und $R_2$ gleich oder verschieden sind und Alkyl oder Alkoxy mit bis zu 8 C-Atomen und einer oder zwei der Substituenten X Fluor und die übrigen Wasserstoff bedeuten.

7. Elektrooptisches Anzeigeelement auf der Basis einer Flüssigkristallzelle, dadurch gekennzeichnet, daß die Flüssigkristallzelle ein Dielektrikum nach Anspruch 6 enthält.

8. Verfahren zur Herstellung der Verbindungen der Formel (I)

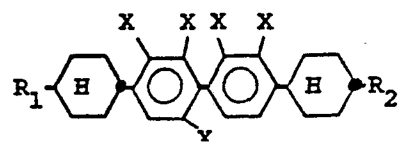

(I)

worin $R_1$ und $R_2$ gleich oder verschieden sind und Alkyl oder Alkoxy mit bis zu 8 C-Atomen, und einer oder zwei der Substituenten X Fluor und die übrigen Wasserstoff bedeuten, dadurch gekennzeichnet, daß man Verbindungen der Formel (II)

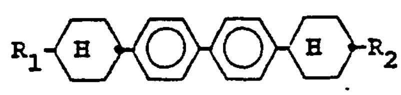

(II)

worin $R_1$ und $R_2$ die vorstehend angegebene Bedeutung besitzen, mit einem Nitrierungsmittel behandelt, die dabei entstehenden Mono- und Dinitroderivate in an sich bekannter Weise abtrennt, zu Mono- bzw. Diaminoverbindungen reduziert, diese in an sich üblicher Weise diazotiert und durch Zugabe von Tetrafluoborat in die Mono- bzw. Di-diazoniumtetrafluoborate überführt, diese thermisch zersetzt und das erhaltene Gemisch von Mono- und Difluorverbindungen der Formel (I) in an sich bekannter Weise in seine Komponenten auftrennt.

**Claims**

1. Compounds of the formula (I)

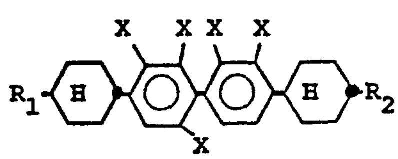

(I)

wherein $R_1$ and $R_2$, which are identical or different, are alkyl or alkoxy having up to 8 C atoms, and one or two of the substituents X are fluorine and the other are hydrogen.

2. Compounds according to Claim 1, of the formula (Ia)

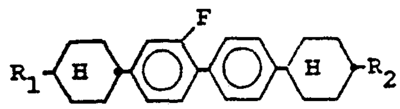

(Ia)

wherein $R_1$ and $R_2$, which are identical or different, are alkyl or alkoxy having up to 8 C atoms.

3. Compounds according to Claim 1, of the formula (Ib)

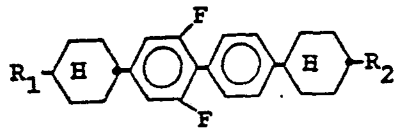

(Ib)

wherein $R_1$ and $R_2$, which are identical or different, are alkyl or alkoxy having up to 8 C atoms.

4. Compounds according to Claim 1, of the formula (Ic)

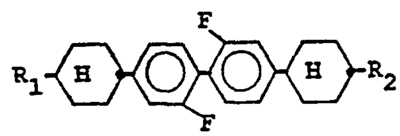

(Ic)

wherein $R_1$ and $R_2$, which are identical or different, are alkyl or alkoxy having up to **8 C atoms.**

5. Use of the compounds of the formula (I)

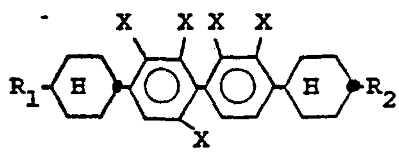

(I)

wherein $R_1$ and $R_2$, which are identical or different, are alkyl or alkoxy having up to 8 C atoms, and one or two of the substituents X are fluorine and the others are hydrogen, as components of liquid-crystalline dielectrics.

6. Liquid-crystalline dielectric for electro-optical display elements, having at least two liquid-crystalline components, characterised in that at least one of these liquid-crystalline components is a compound of the formula (I)

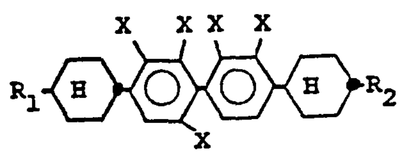

(I)

wherein $R_1$ and $R_2$, which are identical or different, are alkyl or alkoxy having up to 8 C atoms, and one or two of the substituents X are fluorine and the others are hydrogen.

7. Electro-optical display element based on a liquid crystal cell, characterised in that the liquid crystal cell contains a dielectric according to Claim 6.

8. Process for the preparation of compounds of the formula (I)

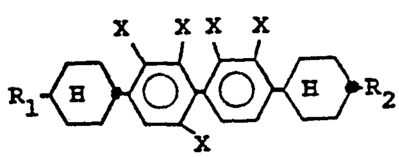

(I)

wherein $R_1$ and $R_2$, which are identical or different, are alkyl or alkoxy having up to 8 C atoms, and one or two of the substituents X are fluorine and the others are hydrogen, characterised in that compounds of the formula (II)

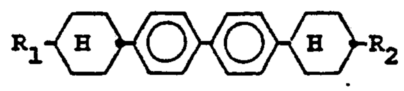

(II)

wherein $R_1$ and $R_2$ have the meanings indicated above, are treated with a nitrating agent, the mononitro and dinitro derivatives thus formed are, in a manner known per se, separated off and reduced to monoamino and diamino compounds, the latter are, in a manner conventional per se, diazotised and converted, by adding tetrafluoborate, into the monodiazonium and didiazonium tetrafluoborates, these are thermally decomposed and the resulting mixture of monofluoro and difluoro compounds of the formula (I) is separated into its components in a manner known per se.

**Revendications**

1. Composés de formule (I)

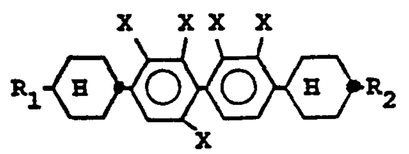

(I)

dans laquelle $R_1$ et $R_2$, identiques ou différents, représentent des groupes alkyle ou alcoxy contenant jusqu'à 8 atomes de carbone, un ou deux des symboles X représentent le fluor et les autres l'hydrogène.

2. Composés selon la revendication 1 de formule (Ia)

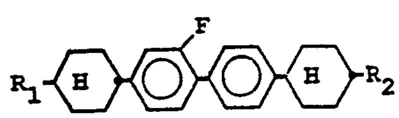

(Ia)

dans laquelle $R_1$ et $R_2$, identiques ou différents, représentent des groupes alkyle ou alcoxy contenant jusqu'à 8 atomes de carbone.

3. Composés selon la revendication 1 de formule (Ib)

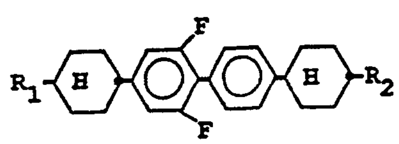

(Ib)

dans laquelle $R_1$ et $R_2$, identiques ou différents, représentent des groupes alkyle ou alcoxy contenant jusqu'à 8 atomes de carbone.

4. Composés selon la revendication 1 de formule (Ic)

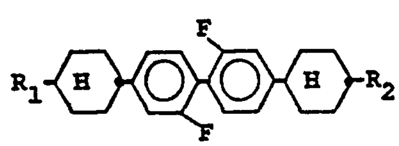

(Ic)

dans laquelle $R_1$ et $R_2$, identiques ou différents, représentent des groupes alkyle ou alcoxy contenant jusqu'à 8 atomes de carbone.

5. Utilisation des composés de formule (I)

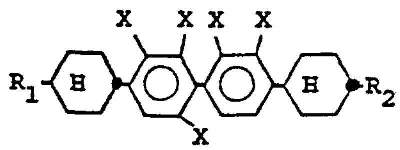

(I)

dans laquelle $R_1$ et $R_2$, identiques ou différents, représentent des groupes alkyle ou alcoxy contenant jusqu'à 8 atomes de carbone, un ou deux symboles X représentent le fluor, les autres l'hydrogène, en tant que composants de diélectriques à cristaux liquides.

6. Diélectrique à cristaux liquides pour éléments d'affichage électro-optiques à au moins deux composants à cristaux liquides, caractérisé en ce que l'un au moins de ces composants à cristaux liquides est un composé de formule (I)

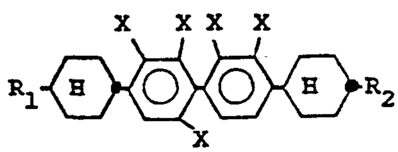

(I)

dans laquelle $R_1$ et $R_2$, identiques ou différents, représentent des groupes alkyle ou alcoxy contenant jusqu'à 8 atomes de carbone, un ou deux des symboles X représentent le fluor et les autres l'hydrogène.

7. Elément d'affichage électro-optique à base d'une cellule à cristaux liquides, caractérisé en ce que la cellule à cristaux liquides contient un diélectrique selon la revendication 6.

8. Procédé de préparation des composés de formule (I)

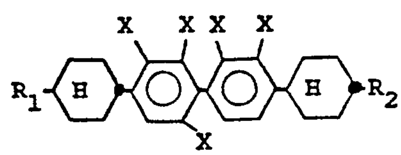

(I)

dans laquelle $R_1$ et $R_2$, identiques ou différents, représentent des groupes alkyle ou alcoxy contenant jusqu'à 8 atomes de carbone, un ou deux des symboles X représentent le fluor et les autres l'hydrogène, caractérisé en ce que l'on traite les composés de formule (II)

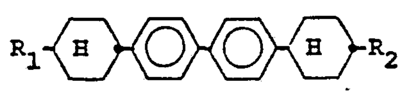

(II)

dans laquelle $R_1$ et $R_2$, ont les significations indiquées ci-dessus, par un agent nitrant, on sépare les dérivés mono- et di-nitrés formés de manière connue en soi, on les réduit respectivement en composés mono- et di-aminés, on diazote ces composés de la manière habituelle et on convertit, par addition d'un tétrafluoborate, en les tétrafluoborates de mono- et di-diazonium respectivement, on décompose ces composés à la chaleur et on sépare le mélange de composés mono- et di-fluorés de formule I ainsi obtenu en ses composants de manière connue en soi.